(19) Europäisches Patentamt
European Patent Office
Office européen des brevets

(11) Veröffentlichungsnummer: **0 195 745**
**B1**

(12) **EUROPÄISCHE PATENTSCHRIFT**

(45) Veröffentlichungstag der Patentschrift: **12.09.90**

(21) Anmeldenummer: **86810118.9**

(22) Anmeldetag: **06.03.86**

(51) Int. Cl.[5]: **C 07 D 471/14,** A 01 N 43/50 // (C07D471/14, 235:00, 221:00, 209:00)

(54) **Heterocyclisch kondensierte Pyridin-Verbindungen als Herbizide.**

(30) Priorität: **12.03.85 CH 1111/85**
**20.12.85 CH 5451/85**

(43) Veröffentlichungstag der Anmeldung:
**24.09.86 Patentblatt 86/39**

(45) Bekanntmachung des Hinweises auf die Patenterteilung:
**12.09.90 Patentblatt 90/37**

(84) Benannte Vertragsstaaten:
**BE CH DE FR GB IT LI NL**

(56) Entgegenhaltungen:
**EP-A-0 041 623**
**EP-A-0 041 624**
**EP-A-0 133 309**
**EP-A-0 161 221**
**EP-A-0 166 907**
**EP-A-0 172 140**
**EP-A-0 183 993**
**US-A-4 041 045**
**US-A-4 404 012**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Dürr, Dieter, Dr.**
**Brändelistalweg 16**
**CH-4103 Bottmingen (CH)**
Erfinder: **Brunner, Hans-Georg, Dr.**
**Wannenstrasse 14**
**CH-4415 Lausen (CH)**
Erfinder: **Szczepanski, Henry, Dr.**
**Bodenmatt**
**CH-4323 Wallbach (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 195 745 B1

**Beschreibung**

Die vorliegende Erfindung betrifft neue Pyridin-Verbindungen mit herbizider und den Pflanzenwuchs regulierender Wirkung, sowie die Herstellung dieser neuen Verbindungen. Ferner betrifft sie Mittel, welche die neuen Imidazolinon Verbindungen enthalten sowie deren Verwendung zur selektiven Bekämpfung von Unkräutern oder zur Regulierung des Pflanzenwuchses.

Bei den neuen Pyridin-Verbindungen handelt es sich insbesondere um Derivate des 3-Isopropyl-3-methyl-3H-imidazo[1',2':1,2]pyrrolo[3,4-b]-pyridin-2,5-dions, entsprechend der Formel I

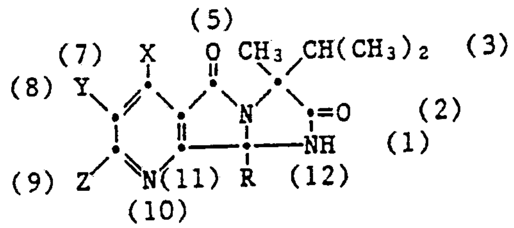

(I)

worin X, Y und Z unabhängig voneinander je Wasserstoff oder eine $C_1$—$C_4$-Alkylgruppe darstellen oder zwei benachbarte Substituenten zusammen auch eine gesättigte oder ungesättigte 3-, 4-gliedrige Alkylenkette resp. Alkenylenkette bilden, die ihrerseits wieder ein bis 4-fach durch $C_1$—$C_4$-Alkyl substituiert sein kann; R eine $C_1$—$C_4$-Nitroalkyl-, die Hydroxyamino-, eine $C_1$—$C_4$-Alkoxyamino, $C_3$—$C_4$-Alkenylamino- oder eine $C_3$—$C_4$-Alkinyloxyaminogruppe oder ein Rest —$PO(OC_1$—$C_4\text{-Alkyl})_2$, —$PO(CH_3)OC_1$—$C_4$-alkyl oder ein Hydrazinorest —$NR_1NR_2R_3$ wobei $R_1$ Wasserstoff oder $C_1$—$C_4$-Alkyl, $R_2$ Wasserstoff, $C_1$—$C_4$-Alkyl oder Benzyl und $R_3$ Wasserstoff, $C_1$—$C_4$-Alkyl, Benzoyl, Carbamoyl, Thiobenzoyl, Thiocarbamoyl, mono- oder di-$C_1$—$C_4$-Alkylcarbamoyl, mono- oder di-$C_1$—$C_4$-Alkylthiocarbamoyl, Anilido, Thioanilido, $C_1$—$C_4$-Alkoxycarbonyl, $C_1$—$C_4$-Alkylcarbonyl, $C_1$—$C_4$-Alkylthiocarbonyl, Benzoyloxy wobei die Phenylringe unsubstituierte oder durch Halogen, $C_1$—$C_4$-Alkyl, $C_1$—$C_4$-Halogenalkyl oder $C_1$—$C_4$-Alkoxy substituiert sein können, oder $R_3$ bedeutet einen zweiten 3H-Imidazo[1',2':1,2]pyrrolo[3,4-b]-pyridin-2,5-dion-12-amino- oder einen Thiazolylcarbamoylrest.

In diesen Definitionen können die Alkyl-, Alkenyl- und Alkylinreste, geradkettig oder verzweigt sein und die Bedeutung von Methyl, Aethyl, Aethylen, Propyl, Propylen, Isopropyl, 1- oder 2-Methyläthylen, Butyl, Butylen, sec. Butyl, 1-Methylpropylen, Isobutyl, 2-Methylpropylen, tert.-Butyl, 2,2-Dimethyläthylen, 1,2-Dimethyläthylen, Allyl, Methallyl, Butenyl, Propagyl, 2-Methylpropinyl oder Butinyl bedeuten.

Halogen bedeutet Fluor, Chlor, Brom oder Iod.

3H-imidazol[1',2':1,2]pyrrolo[3,4-b]-pyridin-2,5-dione sind bekannt. Die EP—A—133 309 beschrieb strukturähnliche uber herbizid schwächer wirksame Derivate. Ausgangsverbindungen der Formel IIa und

IIb sind in der EP—A—41 623 beschrieben. Die U.S. Patentschrift beschreibt verschieden substituierte Verbindungen.

Die Herstellung solcher Verbindungen lässt sich durch folgendes Schema darstellen:

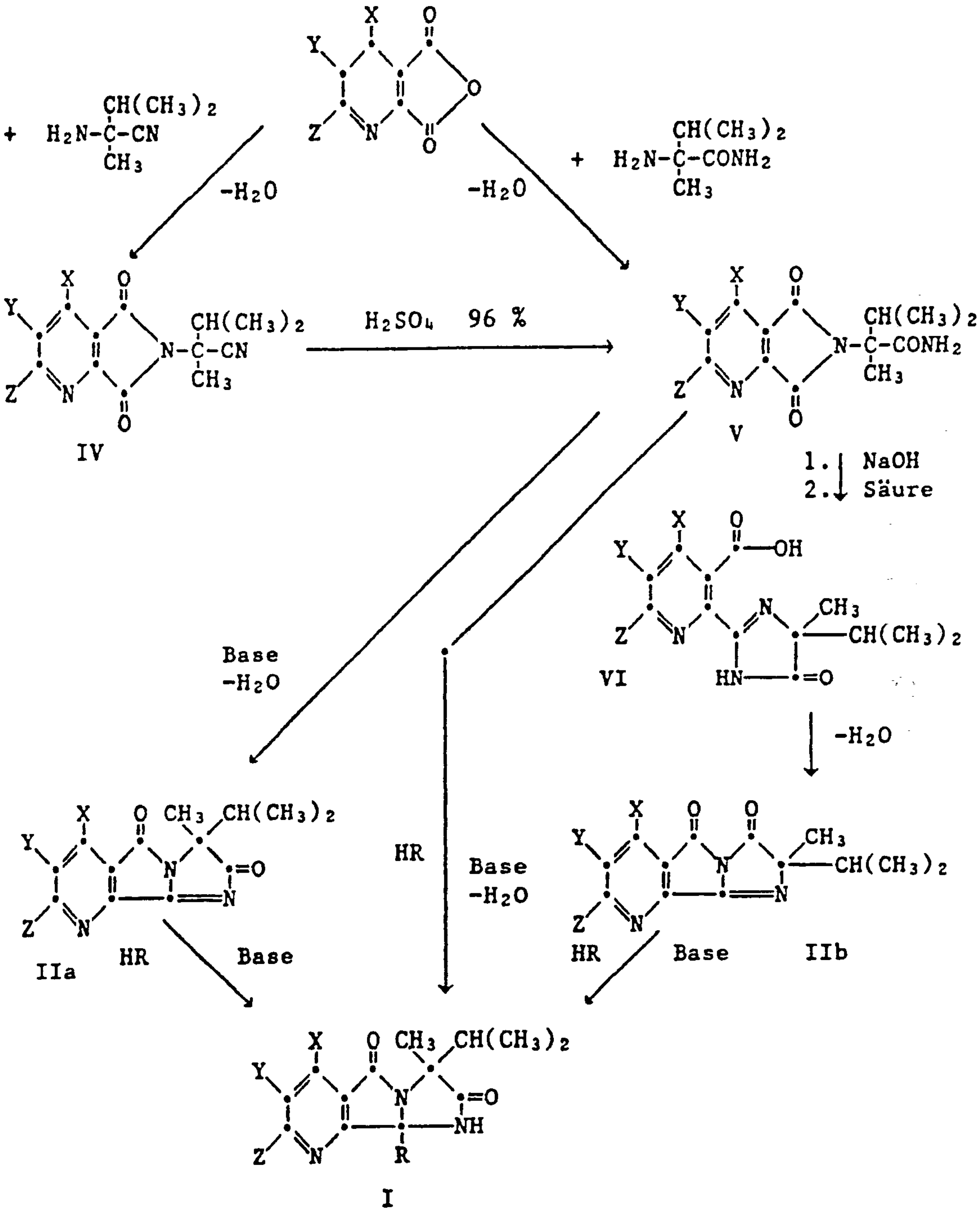

Das erfindungsgemässe Verfahren zur Herstellung der Imidazolinon-Verbindungen der Formel I besteht darin, dass man eine Verbindung der Formel IIa resp. IIb oder V

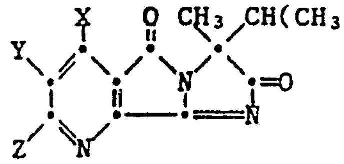 (IIa) 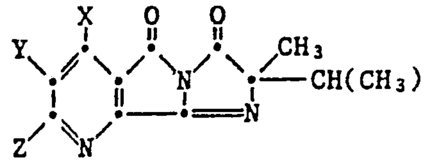 (IIb)

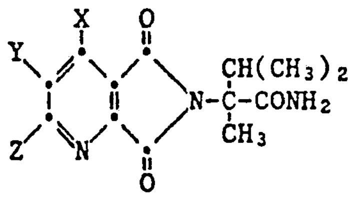 (V)

worin X, Y und Z die unter Formel I gegebenen Bedeutung haben, in einem inerten organischen Lösungs- oder Verdünnungsmittel in Gegenwart einer Base, mit einer Verbindung der Formel III umsetzt,

$$HR \qquad (III)$$

worin R eine der unter der Formel I gegebenen Bedeutungen hat und das entstandene Kondensationsprodukt durch Ansäuern mit einer wässrigen Säure aus dem Reaktionsgemisch isoliert.

Die Verbindungen der Formel IIa, in denen X, Y und Z nicht alle Wasserstoff bedeuten, sind neu und bilden einen Gegenstand dieser, Erfindung.

Bei der Umsetzung wird die Verbindung der Formel III in einem geeigneten Lösungsmittel, wie z.B. Dioxan, Tetrahydrofuran, Acetonitril mit den Ausgangsprodukten der Formel IIa oder IIb zur Verbindung der Formel I umgesetzt. Die Reaktion wird vorzugsweise basisch durchgeführt, kann aber auch sauer katalysiert werden. Das resultierende Produkt wird nach Verdünnen mit Wasser und Ansäuren mit Säuren ausgefällt und abfiltriert oder mit einem Lösungsmittel extrabiert. Die Verbindung der Formel I kann dann nach Verdampfen des Lösungsmittels isoliert werden. Durch Umkristallisation oder Destillation im Hochvakuum fällt das Produkt rein an.

Gewisse Ausgangsprodukte der Formel IIa und IIb sind bekannt andere können nach bekannten Methoden hergestellt werden.

Ein als Ausgangsmaterial benötigtes Pyridin-2,3-dicarbonsäure-α-isopropyl-α-methyl-acetonitril der Formel V kann gemäss dem in der Europäischen Patentanmeldung EP—A—161 221 beschriebenen Verfahren in einfacher Weise hergestellt, indem man ein umgesättigtes Hydrazon mit einem 2-Chlor- oder 2-Brom-N (α-isopropyl-α-methyl-acetonitril)-succinimid kondensiert gemäss dem Schema

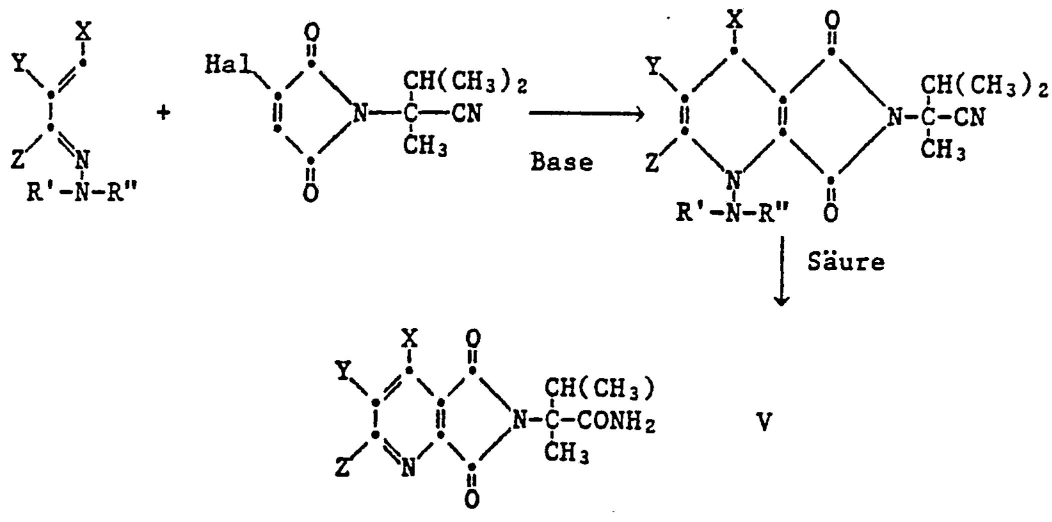

V

In diesen Formeln bedeuten R′ und R″ je Wasserstoff oder $C_1$—$C_4$-Alkyl, Hal Chlor oder Brom während X, Y und Z die unter der Formel I gegebene Bedeutung haben.

Man kann des Ausgangsprodukt der Formel V auch herstellen nach dem in der Europäischen Patentanmeldung No. EP—A—172 140 beschriebenen Verfahren, indem man ein ungesättigtes Hydrazon

mit N-(α-Isopropyl-α-methyl-acetonitril)-succinimid kondensiert. Dabei entsteht ein Tetrahydropyridin-2,3-dicarbonsäureimid, welches durch Behandeln mit Kieselgel und nachheriges Oxidieren an Luft zum Pyridin-2,3-dicarbonsäureimid umgewandelt wird, entsprechend dem Schema:

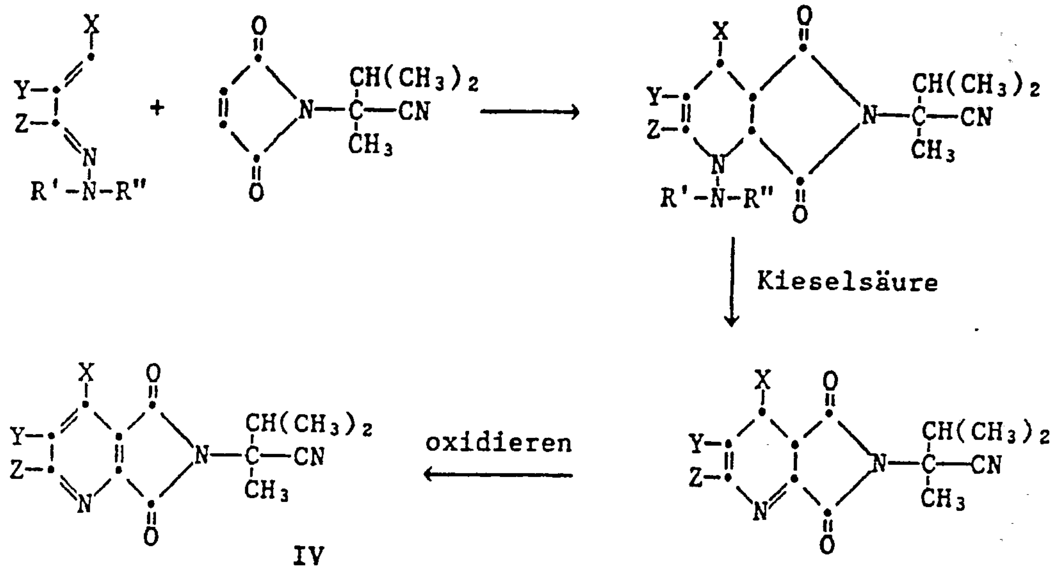


IV

In diesen Formeln bedeuten R' und R'' je Wasserstoff oder $C_1$—$C_4$-Alkyl, X, Y und Z haben die unter der Formel I gegebene Bedeutung.

Wenn man das N-(α-Isopropyl-α-methylacetonitril)-2,3-pyridindicarbonsäureimid der Formel IV zuerst mit konzentrierter Schwefelsäure behandelt, erhält man unter Hydrolyse der Nitrilgruppe das N-(α-Isopropyl-α-methyl-acetamido)-2,3-pyridincarbonsäureimid der Formel V welches unter basischen Bedingungen zum 3-Isopropyl-3-methyl-3H-imidazo[1',2':1,2]pyrrolo[3,4-b]-pyridin-2,5-dion der Formel IIa kondensiert. Man kann es aber auch in Gegenwart einer Base wie z.B. Natronlauge zum 2-(4-Isopropyl-4-methyl-5-oxo-imidazolidin)-nicotinsäurederivat der Formel VI umwandeln, welche sich beim Behandeln mit einem wasserentziehenden Mittel in einem inerten organischen Lösungsmittel unter Verlust eines Wassermoleküls zum Ausgangsprodukt der Formel IIb (2-Isopropyl-2-methyl-3H-imidazo[1',2':1,2]pyrrolo[3,4-b]pyridin 3,5-dion) umwandelt. Nicotinsäuren der Formel VI und ihre Ester sowie deren Herstellung sind z.B. auch in der Europäischen Offenlegungsschrift EP—A—41 623 beschrieben.

Als wasserentziehende Mittel für diese Cyclisation kommen z.B. Kochen am Wasserabscheider, eine molare Menge einer starken Säure z.B. konzentrierter Schwefelsäure oder eines Anhydrides, oder ein wasserabsorbierende Reagentien wie Cyclohexancarbodiimid, Thionylchlorid oder Phosgen in Gegenwart von etwas Dimethylformamid in Frage.

Die Reaktionen werden, soweit sie sich nicht schon bei Raumtemperatur durchführen lassen, bei Temperaturen zwischen 0°C und 200°C durchgeführt, d.h. man erhitzt — falls nötig — bis zum Siedepunkt des Reaktionsgemisches und kühlt wenn nötig mit Eis/Wasser oder Eis/Solebad ab.

Als Basen kommen für diese Kondensationen resp. Hydrolysierungen vor allem anorganische Basen wie Natriumhydroxid, Natriumcarbonat, Calciumhydroxid, Calciumcarbonat, Kaliumhydroxid, Kaliumcarbonat, Ammoniak sowie tertiäre organische Basen wie Triäthylamin in Frage.

Geeignete Lösungsmittel sind z.B. polare, aprotische Lösungsmittel, die alleine oder in Mischungen aus mindestens zwei Lösungsmitteln verwendet werden können. Beispiele sind: Ether wie Dibutylether, Tetrahydrofuran, Dioxan, Methylenglykol, Dimethylethylenglykol, Diethyldiethylenglykol, Dimethyltriethylenglykol, halogenierte Kohlenwasserstoffe wie Methylenchlorid, Chloroform, 1,2-Dichlorethan, 1,1,1-Trichlorethan, 1,1,2,2-Tetrachlorethan, Carnbonsäureester und Lactone wie Essigsäureethylester, Propionsäuremethylester, Benzoesäureethylester, 2-Methoxyethylacetat, γ-Butyrolacton, o-Valerolacton und Pivalolacton, Carbonsäureamide und Lactame wie Formamid, Acetamid, N-Methylformamid, N,N-Dimethylformamid, N,N-Diethylformamid, N-N-Dimethylacetamid, N,N-Diethylacetamid, γ-Butyrolactam, ε-Caprolactam, N-Methylpyrrolidon, N-Acetylpyrrolidon, N-Methylcaprolactam, Tetramethylharnstoff, Hexamethylphosphorsäuretriamid, Sulfoxide wie Dimethylsulfoxid, Sulfone wie Dimethylsulfon, Diethylsulfon, Trimethylensulfon, Tetramethylensulfon, Trimethylamin, Triethylamin, N-Methylpyrrolidin, N-Methylpiperidin, N-Methylmorpholin, substituierte Benzole wie Chlorbenzol, Nitrobenzol, Nitrile wie z.B. Acetonitril.

Bevorzugt als Herbizide oder zur Regulierung des Pflanzenwuchses sind diejenigen Derivate des 3-Isopropyl-3-methyl-3H-imidazo[1',2':1,2]pyrrolo[3,4-b]pyridin-2,5-dions der Formel I, in denen R einen Hydrazinorest bedeutet, besonders

12-Hydrazino-3,8-dimethyl-3-isopropyl-3H-imidazo[1',2':1,2]pyrrolo[3,4-b]pyridin-2,5-dion.
R den Hydroxylaminorest bedeutet, besonders
12-Hydroxylamino-3-isopropyl-3-methyl-3H-imidazo[1',2':1,2]pyrrolo[3,4-b]-pyridin-2,5-dion.
R einen $C_1$—$C_4$-Alkoxyamino- oder $C_3$—$C_4$-Alkenyloxyaminorest bedeutet, besonders
3-Isopropyl-12-methoxyamino-3-methyl-3H-imidazo[1',2':1,2]pyrrolo[3,4-b]pyridin-2,5-dion,
12-Allyloxyamino-8-n-butyl-3-isopropyl-3-methyl-3H-imidazo[1',2':1,2]pyrrolo[3,4-b]pyridin-2,5-dion,
12-Allyloxyamino-3-isopropyl-3-methyl-3H-imidazo[1',2':1,2]pyrrolo[3,4-b]pyridin-2,5-dion und
8-Aethyl-12-allyloxyamino-3-isopropyl-3H-imidazo[1',2':1,2]pyrrolo[3,4-b]pyridin-2,5-dion.
R eine $C_1$—$C_4$-Nitroalkylgruppe bedeutet, besonders
8-Aethyl-3-isopropyl-3-methyl-12-nitromethyl-3H-imidazo[1',2':1,2]pyrrolo[3,4-b]pyridin-2,5-dion.

Die Erfindung betrifft alle diastereomeren und enantiomeren Isomeren der Verbindungen der Formel I.

Die Wirkstoffe der Formel I werden in der Regel mit Aufwandmengen von 0,05 bis 4 kg/ha insbesondere 0,1 bis 1 kg/ha erfolgreich eingesetzt.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei — im Vergleich zu anderen Herbiziden und Wuchsregulatoren — sehr geringen Aufwandmengen.

Die Verbindungen der Formel I haben ausserdem starke pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindung der Formel I werden in unveränderter Form oder vorzugsweise als Mittel zusammen mit den in der Formulierungstechnik üblichen Hilfsmitteln eingesetzt und werden daher z.B. Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. wie den Wirkstoff der Formel I und gegebenenfalls einen festen oder flüssigen Zusatzstoff enthaltenden Mittel, Zubereitungen ode Zusammensetzungen werden in bekannter Weise hergestellt, z.B. durch inniges Vermischen und/oder Vermahlen der wirkstoffe mit Streckmitteln, wie. z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl-oder-äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, absorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignte anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$—$C_{22}$), weide z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8—22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensatonsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome in Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffdatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol Polyäthylenglykol und Octylphenoxypolyäthoxyäthanol erwähnt.

Ferner kommen auch Fettsäureester von Polyäthylensorbitan wie das Polyoxyäthylensorbitan-trioleat in Betracht.

Bei den kationischen Tensiden handelt es sich vor allem um quaternäre Ammiumsalze, welche als N-Substituenten mindestens einen Alkylrest mit 8 bis 22 C-Atomen enthalten und als weitere Substituenten niedrige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"McCutcheon's Detergents and Emulsifiers Annual", MC Publishing Corp., Ridgewood, New Jersey, 1979.

Dr. Helmut Stache, "Tensid Taschenbuch", Carl Hanser Verlag, München/Wien 1981.

Die erfindungsgemässen Zubereitungen enthalten in der Regel 0,1 bis 95%, insbesondere 0,1 bis 80%, Wirkstoff der Forjel I, 1 bis 99,9% eines festen oder flüssigen Zusatzstoffes und 0 bis 25%, insbesondere 0,1 bis 25% eines Tensides.

Insesondere setzen sich bevorzugte Formulierungen folgendermassen zusammen: (% = Gewichtsprozent)

*Emulgierbare Konzentrate:*

| | |
|---|---|
| Wirkstoff der Formel I: | 1 bis 20%, bevorzugt 5 bis 10% |
| oberflächenaktives Mittel: | 5 bis 30%, vorzugsweise 10 bis 20% |
| flüssiges Trägermittel: | 50 bis 94%, vorzugsweise 70 bis 85%. |

*Stäube*

| | |
|---|---|
| Wirkstoff der Formel I: | 0,1 bis 10%, vorzugsweise 0,1 bis 1% |
| festes Trägermittel: | 99,9 bis 90%, vorzugsweise 99,9 bis 99%. |

*Suspensions-Konzentrate:*

| | |
|---|---|
| Wirkstoff der Formel I: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |

*Benetzbare Pulver:*

| | |
|---|---|
| Wirkstoff der Formel I: | 0,5 bis 90%, vorzugsweise 1 bis 80% |
| oberflächenaktives Mittel: | 0,5 bis 20%, vorzugsweise 1 bis 15% |
| festes Trägermittel: | 5 bis 95%, vorzugsweise 15 bis 90%. |

*Granulate*

| | |
|---|---|
| Wirkstoff der Formel I: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1

Herstellung von 3-Isopropyl-3-methyl-8-n-propyl-3H-imidazo[1',2':1,2]pyrrolo[3,4-b]pyridin-2,5-dion (Ausgangsmaterial)

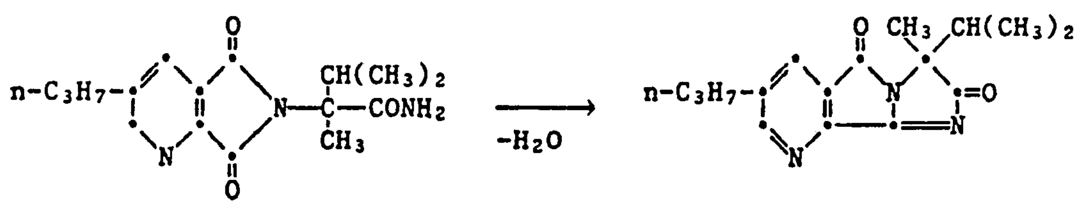


Eine Lösung von 5-n-Propyl-5,7-dihydro-α-isopropyl-α-methyl-5,7-dioxo-6H-pyrrolo-[3,4-b]pyridin-6-acetamid (Smp. 82°C) in 100 ml Toluol wird unter Zugabe von 0,5 g gepulvertem Natriumhydroxid während 2 Stunden am Wasserabscheider unter Rückfluss erhitzt. Nach dem Erkalten filtriert man die Reaktionslösung durch Kieselgel, wäscht mit Essigsäureäthylester nach und dampft das Filtrat ein. Der

Rückstand wird aus Essigsäureäthylester/Petroläther auskristallisiert. Man erhält so 13,1 g der obigen Verbindung welche bei 116°C schmilzt.

In analoger Weise zu diesem Beispiel werden die Ausgangsverbindungen der Tabelle 1 erhalten.

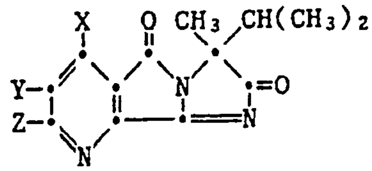

IIa

TABELLE 1

| No | X | Y | Z | phys. Daten |
|---|---|---|---|---|
| 1.01 | H | $nC_3H_7$ | H | Smp. 116° |
| 1.02 | H | $C_2H_5$ | H | Smp. 119—120° |
| 1.03 | H | $n\text{-}C_4H_9$ | H | Smp. 86—87° |
| 1.04 | H | $CH(CH_3)_2$ | H | |
| 1.05 | H | $CH_3$ | H | Smp. 102—106° |
| 1.06 | $C_2H_5$ | $CH_3$ | H | Smp. 128—129° |
| 1.07 | $—(CH{=}CH)_2—$ | — | H | |
| 1.08 | H | $—(CH{=}CH)_2—$ | — | |
| 1.09 | H | $—(CH_2—CH_2)_2—$ | — | |
| 1.10 | $—(CH_2—CH_2)_2—$ | — | H | Smp. 95° |
| 1.11 | $CH_3$ | H | $CH_3$ | |
| 1.12 | H | $—(CH_2—CH_2)_2—$ | — | |
| 1.13 | H | H | $CH_3$ | |
| 1.14 | H | $CH_3$ | $CH_3$ | |
| 1.15 | H | $CH(CH_3)C_2H_5$ | H | |

Beispiel 2

Herstellung von 2-Isopropyl-2-methyl-7-n-propyl-3H-imidazo[1',2':1,2]pyrrolo[3,4-b]-pyridin-3,5-dion (Ausgangsmaterial)

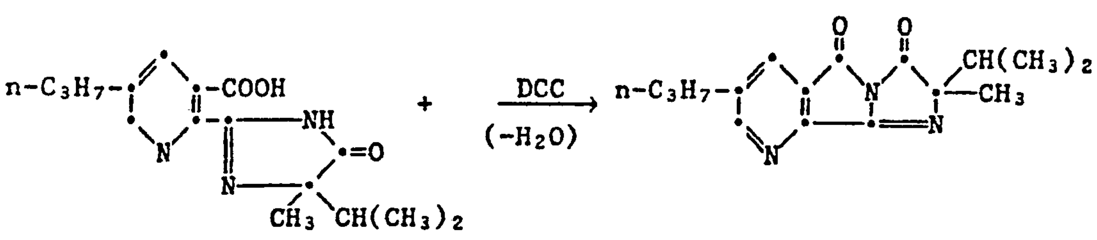


Zu einer Lösung von 11,1 g Dicyclohexylcarbodiimid in 100 ml Methylenchlorid gibt man unter Rühren 15 g (0.05 Mol) 2-(5-Isopropyl-5-methyl)-2-oxo-2-imidazolin-2-yl-5-n-propylnicotinsäure und rührt zwei Stunden bei Raumtemperatur weiter. Dann nutscht man den entstandene Dicyclohexylharnstoff ab und wäscht den Rückstand mit wenig Methylenchlorid nach. Das Filtrat wird eingedampft und der Rückstand aus Essigsäureäthylester/Hexan umkristallisiert. Man erhält so das Titelprodukt in 91%iger Ausbeute (12.1 g) als Kristalle, die bei 132—133° schmelzen.

In analoger Weise zu diesem Beispiel werden die in Tabelle 2 aufgeführten Ausgangsprodukte erhalten.

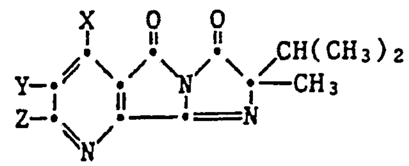

IIb

TABELLE 2

| No | X | Y | Z | phys. Daten |
|---|---|---|---|---|
| 2.01 | H | n-$C_3H_7$ | H | Smp. 132—133° |
| 2.02 | H | $CH(CH_3)_2$ | H | Smp. 145—146° |
| 2.03 | H | $C_2H_5$ | H | Smp. 136—138° |
| 2.04 | H | n-$C_4H_9$ | H | Smp. 116—119° |
| 2.05 | $C_2H_5$ | $CH_3$ | H | |
| 2.06 | $—(CH{=}CH)_2—$ | — | H | |
| 2.07 | $—(CH_2—CH_2)_2—$ | — | H | |
| 2.08 | H | $CH_3$ | H | Smp. 129—131° |

Beispiel 3

Herstellung von 8-Aethyl-3-isopropyl-3-methyl-12-nitromethyl-3H-imidazo[1',2':1,2]pyrrolo[3,4-b]-pyridin-2,5-dion

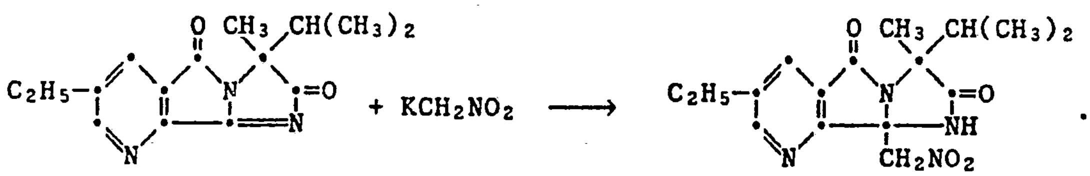

Zu einer Mischung von 6,3 g Kalium-tert.butanolat in 50 ml Tetrahydrofuran gibt man unter Rühren 6 ml Nitromethan. Nach 15 Minuten werden 15 g 8-Aethyl-3-isopropyl-3-methyl-3H-imidazol[1',2':1,2]-pyrrolo[3,4-b]-2,5-dion zugegeben. Nach ca. 10 Stunden Rühren bei Raumtemperatur versetzt man die Lösung mit 2,2 ml Ameisensäure und ca. 500 ml Wasser und extrahiert mit Methylenchlorid. Die organische Phase wird getrocknet, eingedampft und der Rückstand an einer Keiselgelsäule mit Essigester/Hexan 1:1 als Laufmittel gereinigt. Das Endprodukt kristallisiert aus Aceton/Hexan. Ausbeute 8 g, Schmelzpunkt 161—163°.

In analoger Weise zu diesem Beispiel unter sinngemässer Verwendung entsprechender Ausgangsprodukte werden die folgenden in der Tabelle 3 aufgeführten Verbindungen erhalten.

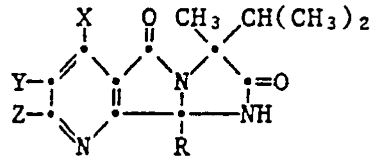

TABELLE 3

| No | X | Y | Z | R | phys. Konstante |
|---|---|---|---|---|---|
| 3.01 | H | H | H | $—CH_2NO_2$ | Smp. 170—175° |
| 3.02 | H | $CH_3$ | H | $—CH_2NO_2$ | Smp. 160—161° |
| 3.03 | H | $n-C_3H_7$ | H | $—CH_2NO_2$ | |
| 3.04 | H | $CH(CH_3)_2$ | H | $—CH_2NO_2$ | |
| 3.05 | H | $n-C_4H_9$ | H | $—CH_2NO_2$ | |
| 3.06 | H | H | $C_2H_5$ | $—CH_2NO_2$ | |
| 3.07 | $—(CH=CH)_2—$ | | H | $—CH_2NO_2$ | |
| 3.08 | H | $—(CH=CH)_2—$ | — | $—CH_2NO_2$ | |
| 3.09 | $—(CH_2—CH_2)_2$ | — | H | $—CH_2NO_2$ | |
| 3.10 | H | H | H | $—CH(CH_3)NO_2$ | Smp. 153—154° |
| 3.11 | H | $CH_3$ | H | $—CH(CH_3)NO_2$ | Smp. 151—152° |
| 3.12 | H | $C_2H_5$ | H | $—CH(CH_3)NO_2$ | |
| 3.13 | H | $—(CH=CH)_2—$ | — | $—CH(CH_3)NO_2$ | |
| 3.14 | H | $C_2H_5$ | H | $—CH(C_2H_5)NO_2$ | Smp. 187—189° |
| 3.15 | H | H | H | $—CH(n-C_3H_7)NO_2$ | |
| 3.16 | H | H | $CH_3$ | $—CH_2NO_3$ | |
| 3.17 | H | $CH_3$ | H | $—CH(C_2H_5)NO_2$ | Smp. 150—151° |
| 3.18 | H | $C_2H_5$ | H | $—CH_2NO_2$ | Smp. 161—163° Beispiel 3 |

Beispiel 4

Herstellung von 12-Hydrazino-3-isopropyl-3-methyl-3H-imidazo[1',2':1,2]pyrrolo[3,4-b]chinolin-2,5-dion

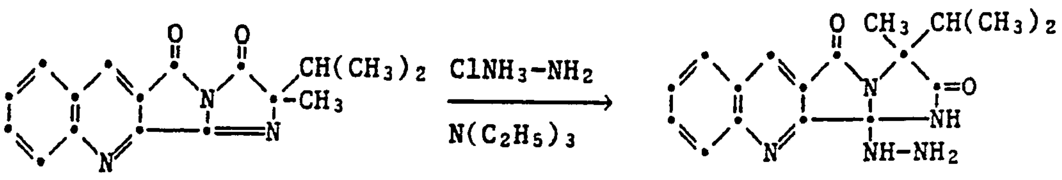


Ein Gemisch von 8,8 g 2-Isopropyl-2-methyl-3H-imidazo[1',2':1,2]pyrrolo[3,4-b]chinolin-pyridin-3,5-dion in 50 ml Tetrahydrofuran wird mit 85 ml Triäthylamin und 2.1 g Hydrazinhydrochlorid versetzt und ca. 30 Stunden lang bei Raumtemperature gerührt. Das Lösungsmittel wird dann eingedampft und der Rückstand in 200 ml Wasser verrührt, wobei das Endprodukt kristallisiert. Nach dem Abnutschen und Trocknen erhält man 6,4 g Titelprodukt vom Schmelzpunkt 280—283°.

Beispiel 5

Herstellung von 3-Isopropyl-3-methyl-(N'-methyl-N'-anilidothiocarbonylhydrazino)-3H-imidazo[1',2':1,2]pyrrolo[3,4-b]pyridin-2,5-dion

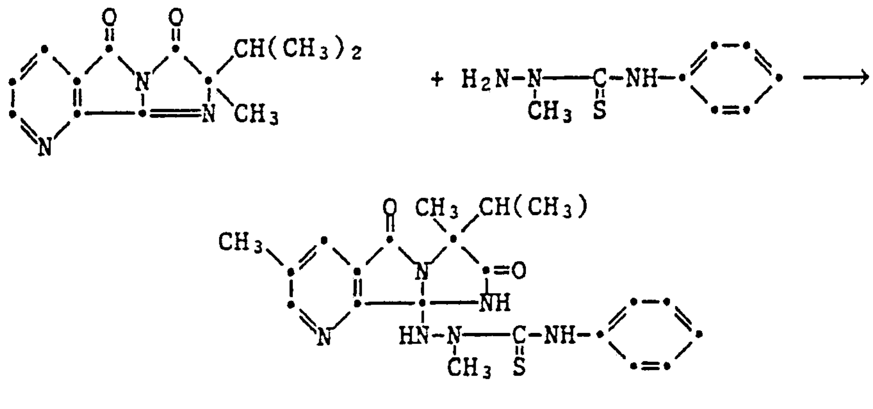

Zu einem Gemisch von 7,3 g 2-Isopropyl-2-methyl-3H-imidazo[1',2':1,2]pyrrolo[3,4-b]-pyridin-3,5-dion in 100 ml Tetrahydrofuran tropft man langsam unter Rühren und 5,4 g 1-Amino-1-methyl-3-phenylthioharnstoff. Nachdem alles zugesetzt ist rührt man 18 Stunden bei Raumtemperatur weiter. Das Reaktionsgemisch wird dann eingedampft und der Rückstand aus Diäthyl-äther kristallisiert. Man erhält so 9 g Titelprodukt vom Schmelzpunkt 162—163°C.

In analoger Weise zu diesen Beispielen werden die in der Tabelle 4 aufgeführt Verbindungen enthalten.

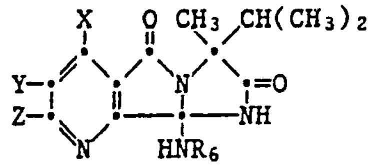

TABELLE 4

| Nr. | X | Y | Z | $R_6$ | phys. Konstante |
|---|---|---|---|---|---|
| 4.01 | H | H | H | $NH_2$ | Smp. 195—225° |
| 4.02 | H | H | H | $NHCOOCH_3$ | Smp. 112° |
| 4.03 | H | H | H | 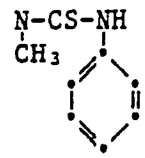 | Smp. 162—163° Beispiel 9 |
| 4.04 | H | H | H | 3-Isopropyl-3-methyl-3H-imidazo[1′,2′:1,2]pyrrolo-[3,4-b]pyridin-2,5-dion-12-amino | Smp. 227 Zers. |
| | | | | | Smp. 197—198° |
| 4.05 | H | H | H | 4-Methyl-thiazol-5-yl-carbamoyl | |
| 4.06 | H | $CH_3$ | H | $NH_2$ | |
| 4.07 | H | $CH_3$ | H | 3,8-Dimethyl-3-isopropyl-3H-imidazo[1′,2′:1,2]pyrrolo-[3,4-b]pyridin-2,5-dion-12-amino | Smp. 207—208° |
| 4.08 | H | $C_2H_5$ | H | $NH_2$ | |
| 4.09 | H | $-(CH_2{=}CH)_2-$ | | $NH_2$ | Smp. 280—283° Beispiel 8 |
| 4.10 | H | $-(CH_2{=}CH)_2-$ | | $NH-CO-OCH_3$ | |
| 4.11 | H | $-(CH_2{=}CH)_2-$ | | $NH-CO-CH_3$ | |
| 4.12 | H | $CH_3$ | H | $NH-CO-CH_3$ | |
| 4.13 | H | $C_2H_5$ | H | $N(CH_3)_2$ | |
| 4.14 | H | $CH_3$ | H | $-N(CH_3)_2$ | |

Beispiel 6

Herstellung von 12-Hydroxylamino-3-isopropyl-3-methyl-3H-imidazo[1′,2′:1,2]pyrrolo[3,4-b]pyridin-2,5-dion

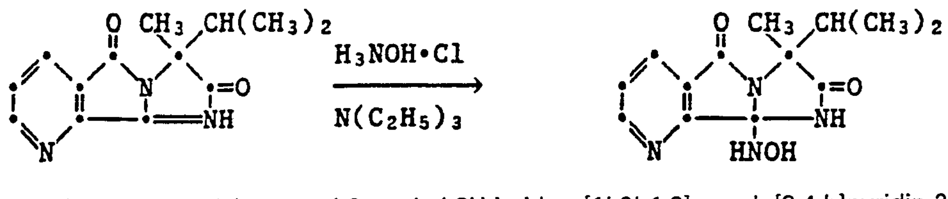


Ein Gemisch von 7,3 g 3-Isopropyl-3-methyl-3H-imidazo[1′,2′:1,2]pyrrolo[3,4-b]pyridin-2,5-dion, 100 ml Acetonitril, 2,3 g Hydroxylamin-Hydrochlorid und 7 ml Triäthylamin wird bei Raumtemperatur gerührt. Nach ca. 30 Stunden dampft man das Lösungsmittel unter vermindertem Druck ab und gibt zum Rückstand

Wasser, wo das Titelprodukt nach kurzem Rühren kristallin und analysenrein ausfällt. Ausbeute nach Abnutschen und Trocknen 5 g Schmelzpunkt 163,3—164,3° unter Zersetzen.

Analog zu diesem Beispiel werden die in der Tabelle 5 aufgeführten Verbindungen erhalten.

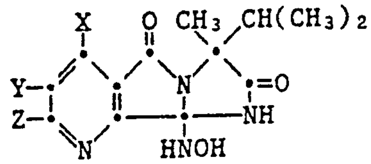

TABELLE 5

| Nr. | X | Y | Z | phys. Konstante |
|---|---|---|---|---|
| 5.01 | H | H | H | Smp. 163—164° Z Beispiel 10 |
| 5.02 | H | $CH_3$ | H | Smp. 159—161° |
| 5.03 | H | $C_2H_5$ | H | |
| 5.04 | H | $C_3H_7n$ | H | |
| 5.05 | H | $CH(CH_3)_2$ | H | |
| 5.06 | $CH_3$ | $C_4H_9n$ | H | |
| 5.07 | H | $-(CH=CH)_2$ | | Smp. 196° Zers. |
| 5.08 | H | $-(CH_2-CH_2)_2$ | | |
| 5.09 | $CH_3$ | H | H | |
| 5.10 | H | $CH_3$ | $CH_3$ | |
| 5.11 | H | $C_2H_5$ | $CH_3$ | |
| 5.12 | $-(CH=CH)_2$ | | | |

Beispiel 7

Herstellung von 12-Allyloxyamino-8-n-butyl-3-isopropyl-3-methyl-3H-imidazo[1′,2′:1,2]pyrrolo[3,4-b]-pyridin-2,5-dion

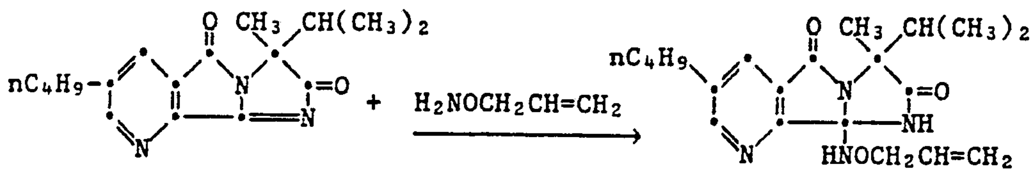


Zu einer gerührten Mischung von 5 g 8-n-Butyl-3-isopropyl-3-methyl-3H-imidazo[1′,2′:1,2]pyrrolo[3,4-b]pyridin-2,5-dion in 50 ml Dioxan gibt man 2,95 ml Triäthylamin und 2,3 g O-Allylhydroxylamin-Hydrochlorid. Nach einigen Stunden Rühren bei Raumtemperatur verdünnt man das Reaktionsgemisch mit ca. 500 ml Wasser und extrahiert mit Acetylacetat. Die organische Phase wird getrocknet, eingedampft und der Rückstand aus Aceton/Wasser umkristallisiert. Man erhält so in guter Ausbeute 5,3 g) das Titelprodukt, welches bei 143—145° schmilzt.

Beispiel 8

Herstellung von 3-Isopropyl-3-methyl-12-allyloxyamino-3H-imidazo[1′,2′:1,2]pyrrolo[3,4-b]pyridin-2,5-dion

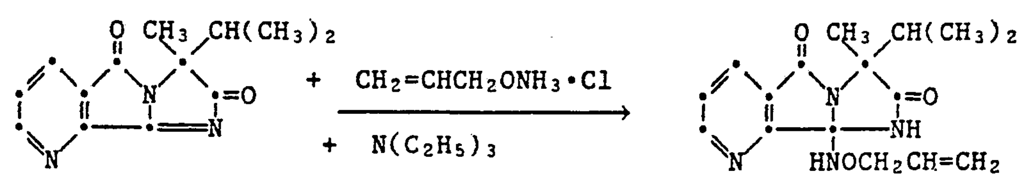


Ein Gemisch von 7,3 g 3-Isopropyl-3-methyl-3H-imidazo[1′,2′:1,2]pyrrolo[3,4-b]-pyridin-2,5-dion, 50 ml

Dioxan, 3,4 g O-Allylhydroxylamin-Hydrochlorid und 4,4 ml Triäthylamin wird während ca. 15 Stunden bei Raumtemperatur gerührt. Dann giesst man di entstandene Lösung auf ca. 500 ml Wasser und extrahiert mit Aether. Die Aetherextrakte werden mit Wasser gewaschen, über Natriumsulfat getrocknet und eingeengt. Der Rückstand wird aus Methylenchlorid/Petroläther umkristallisiert. Die Ausbeute an reinem Titelprodukt beträgt 4,9 g. Der Schmelzpunkt ist 122—125°.

In analoger Weise zu den Beispielen 7 und 8 werden unter sinngemässer Verwendung entsprechender Ausgangsmaterialien die in der Tabelle 6 aufgeführten Verbindungen erhalten.

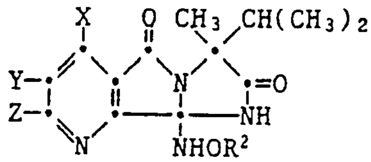

TABELLE 6

| Nr. | X | Y | Z | $R^2$ | phys. Daten |
|---|---|---|---|---|---|
| 6.01 | H | $C_2H_5$ | H | $CH_2CH{=}CH_2$ | Smp. 198—200 |
| 6.02 | H | H | H | $CH_2CH{=}CH_2$ | Smp. 122—125<br>Beispiel 12 |
| 6.03 | H | $nC_4H_9$ | H | $CH_2CH{=}CH_2$ | Smp. 143—145°<br>Beispiel 11 |
| 6.04 | H | H | H | $CH_3$ | Smp. 191—193° |
| 6.05 | H | $CH_3$ | H | $CH_3$ | |
| 6.06 | H | $C_2H_5$ | H | $CH_3$ | Smp. 187—189°<br>Zers. |
| 6.07 | H | H | H | $C_2H_5$ | Smp. 135—136° |
| 6.08 | H | H | H | $C_3H_7$-n | |
| 6.09 | H | H | H | $C_4H_9$-n | |
| 6.10 | H | $C_2H_5$ | H | $C_2H_5$ | |
| 6.11 | H | $CH_3$ | H | $CH(CH_3)_2$ | |
| 6.12 | H | —CH=CH— | | $CH_3$ | |
| 6.13 | —(CH=CH)— | | H | $CH_3$ | |
| 6.14 | H | $—(CH{=}CH)_2—$ | | $CH_2CH{=}CH_2$ | |
| 6.15 | H | $CH_3$ | H | $C_2H_5$ | Smp. 192—194° |
| 6.16 | H | H | H | 2-Chlorbenzyl | Smp. 193—194° |

Beispiel 9

Herstellung von 12-Diethylphosphonyl-3-isopropyl-3-methyl-3H-imidazo-[1',2':1,2]pyrrolo[3,4-b]-pyridin-2,5-dion

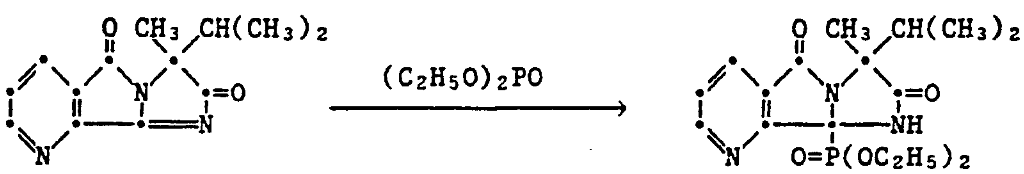


Ein Gemisch von 6 g 3-Isopropyl-3-methyl-3H-imidazo[1',2':1,2]pyrrolo-[3,4-b]pyridin-2,5-dion und 50

ml Diäthylphosphit werden während auf 3 Stunden 100°C erwärmt, und anschliessend am Rotationsverdampfer eingeengt. Der Rückstand wird aus Aceton/Wasser und Methylenchlorid/Hexan umkristallisiert. Die Ausbeute an Titelprodukt beträgt 3 g, Schmelzpunkt 190—194°.

In analoger Weise zum Beispiel 9 werden unter sinngemässer Verwendung entsprechender Ausgangsprodukte die in der Tabelle 7 aufgeführten Verbindungen erhalten.

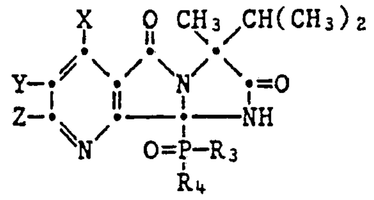

TABELLE 7

| Nr. | X | Y | Z | $R_3$ | $R_4$ | phys. Daten |
|---|---|---|---|---|---|---|
| 7.01 | H | H | H | $OC_2H_5$ | $OC_2H_5$ | Smp. 190—194° |
| 7.02 | H | H | H | $OCH_3$ | $OCH_3$ | Smp. 140—143° |
| 7.03 | H | H | H | $CH_3$ | $OCH_2CH(CH_2)_2$ | Harz |
| 7.04 | H | H | H | $OCH(CH_3)_2$ | $OCH(CH_3)_2$ | Smp. 165+70° Zers. |
| 7.05 | H | $C_2H_5$ | H | $OC_2H_5$ | $OC_2H_5$ | |
| 7.06 | H | $C_2H_5$ | H | $OCH_3$ | $OCH_3$ | |
| 7.07 | H | $C_2H_5$ | H | $OCH(CH_3)_2$ | $OCH(CH_3)_2$ | |
| 7.08 | H | $CH_3$ | H | $OCH_3$ | $OCH_3$ | |
| 7.09 | H | $CH_3$ | H | $OC_2H_5$ | $OC_2H_5$ | |
| 7.10 | H | $CH_3$ | H | $OCH(CH_3)_2$ | $OCH(CH_3)_2$ | |

Formulierungsbeispiele:

Beispiel 10

Formulierungsbeispiele für Wirkstoffe der Formel I (% = Gewichtsprozent)

| a) Spritzpulver | a) | b) | c) |
|---|---|---|---|
| Wirkstoff gemäss Tabellen 3—7 | 20% | 60% | 0,5% |
| Na-Ligninsulfonat | 5% | 5% | 5% |
| Na-Laurylsulfat | 3% | — | — |
| Na-Diisobutylnaphthalinsulfonat | — | 6% | 6% |
| Octylphenolpolyäthylenglykoläther (7—8 Mol AeO) | — | 2% | 2% |
| Hochdisperse Kieselsäure | 5% | 27% | 27% |
| Kaolin | 67% | — | — |
| Natriumchlorid | — | — | 59,5% |

Der Wirkstoff wird mit den Zusatzstoffen gut vermischt und in einer geeigneten Mühle gut vermahlen. Man erhält Spritzpulver, die sich mit Wasser zu Suspension jeder gewünschten Konzentration verdünnen lassen.

| b) Emulsions-Konzentrat | a) | b) |
|---|---|---|
| Wirkstoff gemäss Tabellen 3—7 | 10% | 1% |
| Octylphenolpolyäthylenglykoläther (4—5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesers Konzentrat können durch Verdünnen mit Wasser Emulsionen jeder gewünschten Konzentration hergestellt werden.

| c) Stäubemittel | a) | b) |
|---|---|---|
| Wirkstoff gemäss Tabellen 3—7 | 0,1% | 1% |
| Talkum | 99,9% | — |
| Kaolin | — | 99% |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff gemäss Tabellen 3—7 | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff gemäss Tabellen 3—7 | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspension-Konzentrat | a) | b) |
|---|---|---|
| Wirkstoff gemäss Tabellen 3—7 | 40% | 5% |
| Aethylenglykol | 10% | 10% |
| Nonylphenolpolyäthylenglyköläther (15 Mol AeO) | 6% | 1% |
| Na-Ligninsulfonat | 10% | 5% |
| Carboxymethylcellulose | 1% | 1% |
| 37%iger wässrige Formaldehyd-Lösung | 0,2% | 0,2% |
| Silikonöl in Form einer 75%igen wässrigen Emulsion | 0.8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff gemäss Tabellen 3—7 | 5% |
| Isopropylamin | 1% |
| Octylphenolpolyäthylenglyköläther (78 Mol AeO) | 3% |
| Wasser | 91% |

Biologische Beispiele:

Beispiele 11

Pre-emergente Herbizid-Wirkung

Im Gewächsaus wird unmittelbar nach der Einsaat der Versuchspflanzen in Saatschalen die Erdoberfläche mit einer wässrigen Dispersion der Wirkstoffe, erhalten aus einem 25%igen Emulsionskonzentrat behandelt. Es werden Konzentrationen von 4 kg Wirksubstanz/Hektar angewendet. Die Saatschalen werden im Gewächshaus bei 22—25°C und 50—70% relativer Luftfeuchtigkeit gehalten und der Versuch nach 3 Wochen ausgewertet und die Resultate nach folgender Notenskala ausgeweitet.

1 Pflanze hat nicht gekeimt oder ist eingegangen
2—3 sehr starke Schäden
4 starke Schäden
5 mittlere Schäden, die Pflanzen sind verkümemert
6 Schäden, die Pflanze kann regenerieren
7—8 leichte Schäden
9 normales Wachstum, wie das unbehandelter Pflanzen

In diesem Versuch zeigten die Verbindungen der Tabellen 3—7 starke Herbizidwirkung. Einige Resultate sind in der Tabelle 8 zusammengefasst.

TABELLE 8

| Verb. Nr. | Avena | Setaria | Sinapsis | Stellaria |
|---|---|---|---|---|
| 3.06 | 4 | 2 | 2 | 2 |
| 6.01 | 2 | 1 | 2 | 2 |
| 6.02 | 2 | 1 | 2 | 2 |
| 6.03 | 7 | 3 | 3 | 3 |

Beispiele 12

Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°—26°C und 45—60% relativer Luftfeuchtigkeit gehalten. 15 Tage nach der Behandlung wird der Versuch nach der obigen Notenskala ausgewertet. Auch in diesem Versuch zeigen die Verbindungen der Tabellen 3—7 starke bis sehr starke Herbizidwirkung. Einige Resultate sind in der Tabelle 9 zusammengefasst.

TABELLE 9

| Verb. No. | Avena | Setaria | Lolium | Solanum | Sinapsis | Stellaria |
|---|---|---|---|---|---|---|
| 3.06 | 4 | 3 | 4 | 1 | 2 | 2 |
| 6.01 | 5 | 4 | 4 | 1 | 2 | 2 |
| 6.02 | 1 | 2 | 2 | 1 | 1 | 2 |

Beispiel 13

Herbizidwirkung vor dem Auflaufen der Pflanzen

Kunstofftöpfe werden mit expandiertem Vermiculit (Dichte: 0,135 g/cm$^3$, Wasserabsorptionsvermögen: 0,565 1/1) gefüllt. Nacn dem Sättigen das nicht absorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät. Nasturtium offcinalis, Agrostic tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70% gehalten. Während der Keimphase von 4 bis 6 Tagen werden die Töpfe zur Erhöhung der örtlichen Luftfeuchtigkeit mit lichtdurchlässigem Material abgedeckt und mit deionoisiertem Wasser begossen. Nach dem 5. Tag wird dem Giesswasser 0,5% eines handelsüblichen Flüssigdüngers (®Greenzit) zugesetzt. 12 Tage nach der Aussaat wird der Versuch ausgewertet und die Wirkung auf die Versuchspflanzen nach dem in Beispiel 11 gegebenen Massstab bewertet.

Die Resultate sind in der Tabelle 10 zusammengefasst:

TABELLE 10

| Verb. No. | Aufwand Menge ppm | Nasturtium officinalis | Agrostis tenuis | Stellaria media | Digitaria sang. |
|---|---|---|---|---|---|
| 3.06 | 100 | 1 | 1 | 1 | 1 |
| | 10 | 2 | 2 | 2 | 2 |
| | 1 | 3 | 3 | 3 | 3 |
| | 0.1 | 4 | 5 | 5 | 6 |
| 6.01 | | 2 | 2 | 2 | 2 |
| 6.02 | 100 | 2 | 2 | 2 | 2 |
| | 10 | 3 | 3 | 4 | 4 |
| 6.03 | 100 | 2 | 2 | 2 | 2 |
| | 10 | 3 | 4 | 7 | 7 |

Beispiel 14

Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3—5 mm). Die Applikation erfolgt 3 Tage nach der Saat mit einer wässrigen Emulsion der Prüfsubstanzen durch eine Spritzüng auf die Gefässe. Die Dosis entspricht einer Wirkstoffmenge von 4 kg AS pro Hekter (Spritzbrühmenge = 550 1/ha. Die Pflanzenbecher werden dann im Gewächshaus unter optimalen Wachstumsbedingungen für die Reisunkräuter aufgestellt, d.h. bei 25°—30°C und hoher Luftfeuchtigkeit. Die Auswertung der Versuche findet 3 Wochen nach Applikation statt.

Die Bonitur erfolgt nach im Beispiel 11 gegebenen linearen Notenskala.

Die Resultate sind in der Tabelle 11 zusammengefasst:

TABELLE 11

| Verb. No. | Echinochloa crus galli | Monocharia vag. |
|---|---|---|
| 3.06 | 2 | 1 |
| 6.01 | 2 | 1 |
| 6.02 | 2 | 1 |
| 6.03 | 3 | 1 |

Beispiel 15

Wuchshemmung bei tropischen Bodenbedecker-Leguminosen (cover crops)

Die Versuchspflanzen (Centrosema plumieri und Centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70% relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Tabellen 3—7 in Aufwandmengen von 50—3000 g/ha behandelten Pflanzen eine deutliche Reduktion des Neuzuwachses (weniger als 20% des Neuzuwachses bei unbehandelten Kontrollpflanzen), ohne dass dabei die Versuchspflanzen geschädigt wurden.

Beispiel 16

Wuchsregulierung an Sojabohnen

In Kunststoffbehältern mit einem Erde-Torf-Sandgemisch im Verhältnis 6:3:1 werden Sojabohnen der

Sorte "Hark" angesät und in eine Klimakammer gegeben. Durch optimale Temperaturewahl, Beleuchtung, Düngerzugabe und Bewässerung entwickeln sich die Pflanzen nach ca. 5 Wochen bis zum 5—6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Tabellen 3—7 eine merklicher Erhöhung der Anzahl und des Gewichts der Schoten im Haupttrieb.

Beispiel 17

Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Tabellen 3—7 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60—90% der Kontrolle), sowie teilweise eine Zunahme der Stengeldurchmasser auf.

Beispiel 18

Wuchshemmung bei Gräsern

In Kunststoffschalen mit Erde-Torf-Sand-Gemisch (6:3:1) werden die Grräsr Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabellen 3—7 besprüht. Die Wirkstoffmenge beträgt umgerechnet bis zu 500 g Aktivsubstanz pro Hektar. 21 Tage nach Applikation wird das Wachstum der Gräser beurteilt.

Die geprüften Verbindungen der Tabellen 3—7 bewirken eine Reduzierung des Neuzuwachses von um 10—30% im Vergleich zur unbehandelten Kontrolle.

Beispiel 19

Desiccations- und Defoliationswirkung

In einem Gewächshaus wurden Baumwollpflanzen de Sorte Deltapine in Tontöpfen angezogen. Nach abgeschlossener Kapselbildung wurden sie mit einer wässerigen Zubereitungen eines Wirkstoffes der Tabellen 3—7 in einer Aufwandmenge, die 1,2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Desiccation (% Austrocknung der an der Pflanze verbleibenden Blätter).

In diesem Versuch beliessen die geprüften Verbindungen der Tabellen 3—7 bei Aufwandmengen von 0,6 und 1,2 kg/ha nach 7 Tagen bloss noch wenige vertrocknete Blätter auf den Pflanzen (>80% Blattfall und Austrocknung). Die beste Wirkung zeigte die Verbindung 3.06.

**Patentansprüche**

1. Pyridin-Verbindungen der Formel I

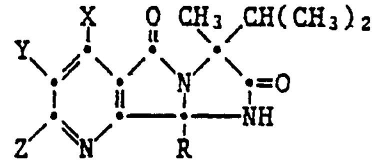

(I)

worin X, Y und Z unabhängig voneinander je Wasserstoff oder eine $C_1$—$C_4$-Alkylgruppe oder zwei benachbarte Substituenten zusammen auch eine gesättigte oder ungesättigte 3-, 4-gliedrige Alkylenkette resp. Alkenylenkette bilden, die ihrerseits wieder ein bis 4-fach durch $C_1$—$C_4$-Alkyl substituiert sein kann; R eine $C_1$—$C_4$-Nitroalkyl- die Hydroxyamino-, eine $C_1$—$C_4$-Alkoxyamino, $C_3$—$C_4$-Alkenyloxyaminogruppe $C_3$—$C_4$-Alkinyloxyaminogruppe oder einen Rest —$PO(OC_1$—$C_4\text{-Alkyl})_2$, —$PO(CH_3)OC_1$—$C_4$-alkyl oder eine Hydrazinorest —$NR_1NR_2R_3$ wobei $R_1$ Wasserstoff oder $C_1$—$C_4$-Akyl, $R_2$ Wasserstoff, $C_1$—$C_4$-Alkyl oder Benzyl und $R_3$ Wasserstoff, $C_1$—$C_4$-Alkyl, Benzoyl, Carbamoyl, Thiobenzoyl, Thiocarbamoyl, mono-oder di-$C_1$—$C_4$-Alkylcarbamoyl, mono-oder di-$C_1$—$C_4$-Alkylthiocarbamoyl, Anilido, Thioanilido, $C_1$—$C_4$-Alkoxycarbonyl, $C_1$—$C_4$-Alkylcarbonyl, $C_1$—$C_4$-Alkylthiocarbonyl, Benzoyloxy wobei die Phenylringe unsubstituierte oder durch Halogen, $C_1$—$C_4$-Alkyl, $C_1$—$C_4$-Halogenalkyl oder $C_1$—$C_4$-Alkoxy substituiert sein können, oder $R_3$ bedeutet einen zweiten 3H-Imidazo[1',2':1,2]pyrrolo[3,4-b]pyridin-2,5-dion-12-yl-rest oder einen Thiazolylcarbamoylrest.

2. Verbindung gemäss Anspruch 1 in denen R den Hydrazinorest darstellt, während Z, Y und Z die im Anspruch 1 gegebene Bedeutung haben.

3. 12-Hydrazino-3,8-dimethyl-3-isopropyl-3H-imidazo[1′,2′:1,2]pyrrolo[3,4-b]pyridin-2,5-dion gemäss Anspruch 1.

4. Verbindungen gemäss Anspruch 1 in denen R den Hydroxylaminorest darstellt, während X, Y und Z die im Anspruch 1 gegebene Bedeutung haben.

5. 12-Hydroxylamino-3-isopropyl-3-methyl-3H-imidazo[1′,2′:1,2]pyrrolo-[3,4-b]pyridin-2,5-dion gemäss Anspruch 1.

6. Verbindungen gemäss Anspruch 1 in denen R einen $C_1$—$C_4$-Alkoxyamino- oder $C_3$—$C_4$-Alkenyloxyaminorest darstellt, während X, Y und Z die im Anspruch 1 gegebene Bedeutung haben.

7. 3-Isopropyl-12-methoxyamino-3-methyl-3H-imidazo[1′,2′:1,2]pyrrolo[3,4-b]pyridin-2,5-dion gemäss Anspruch 1.

8. 12-Allyloxyamino-8-n-butyl-3-isopropyl-3-methyl-3H-imidazo[1′,2′:1,2]pyrrolo[3,4-b]pyridin-2,5-dion gemäss Anspruch 1.

9. 12-Allyloxyamino-3-isopropyl-3-methyl-3H-imidazo[1′,2′:1,2]pyrrolo[3,4-b]pyridin-2,5-dion gemäss Anspruch 1.

10. 8-Aethyl-12-allyloxyamino-3-isopropyl-3-methyl-3H-imidazo[1′,2′:1,2]pyrrolo[3,4-b]pyridin-2,5-dion gemäss Anspruch 1.

11. Verbindungen gemäss Anspruch 1 in denen R einen $C_1$—$C_4$-Nitroalkylrest darstellt, während X, Y und Z die im Anspruch 1 gegebene Bedeutung haben.

12. 8-Aethyl-3-isopropyl-3-methyl-12-nitromethyl-3H-imidazo[1′,2′:1,2]pyrrolo[3,4-b]pyridin-2,5-dion gemäss Anspruch 1.

13. Verfahren zur Herstellung der Verbindungen der Formel I, Anspruch 1, dadurch gekennzeichnet, dass man eine Verbindung der Formel IIa resp. IIb

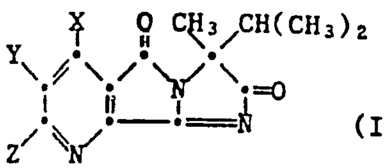
(IIa)

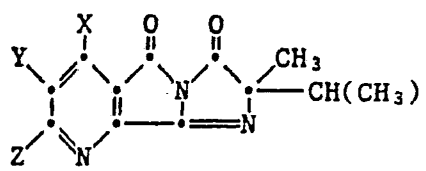
(IIb)

worin X, Y und Z die im Anspruch 1 gegebene Bedeutung haben, in einem inerten organischen Lösungs- oder Verdünnungsmittel in Gegenwart einer Base, mit einer Verbindung der Formel III umsetzt,

HR (III)

worin R eine der im Anspruch 1 gegebenen Bedeutungen hat und das entstandene Kondensationsprodukt durch Ansäuern mit einer wässrigen Säure aus dem Reaktionsgemisch isoliert.

14. Verfahren zur Herstellung der Verbindungen der Formel I, Anspruch 1, dadurch gekennzeichnet, dass man eine Verbindung der Formel V

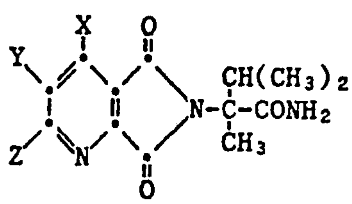
(V)

worin X, Y und Z die im Anspruch 1 gegebenen Bedeutung haben, zuerst in einem inerten organischen Lösungs- oder Verdünnungsmittel unter wasserentziehenden Bedingungen kocht, dann in Gegenwart einer Base, mit einer Verbindung der Formel III umsetzt,

HR (III)

worin R eine der im Anspruch 1 gegebenen Bedeutungen hat und das entstandene Kondensationsprodukt durch Ansäuern mit einer wässrigen Säure aus dem Reaktionsgemisch isoliert.

15. Ein herbizides oder pflanzenwachsregulierdendes Mittel, dadurch gekennzeichnet, dass en neben Träger- und/oder anderen Zuschlagstoffen als Wirkstoff mindestens eine Verbindung gemäss Anspruch 1 enthält.

16. Die Verwendung eines Wirkstoffes gemäss Anspruch 1, oder einen solchen enthaltendes Mittel zur Bekämpfung unerwünschten Pflanzenwachstums.

17. Die Verwendung eines Wirkstoffes gemäss Anspruch 1 oder einem solchen enthaltendes Mittel zur Hemmung des Pflanzenwachstums.

18. Die Verwendung eines Wirkstoffes gemäss Anspruch 1, oder einen solchen enthaltendes Mittel zur Beeinflussung des Pflanzenwachstums zum Zweck der Ertragssteigerung.

19. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge einer Imidazolinon-Verbindung der Formel I, Anspruch 1 oder eines eine solche Verbindung enthaltendes Mittel behandelt.

20. Verfahren zur Unterdrückung des Pflanzenwachstums über das 2-Blattstadium hinaus, dadurch gekennzeichnet, dass man die Pflanzen während ihres Wachstums mit einer wirksamen Menge einer Imidazolinon-Verbindung der Formel I Anspruch 1 oder eines einen solchen Wirkstoff enthaltenden Mittels behandelt.

**Revendications**

1. Dérivé de la pyridine de formule I

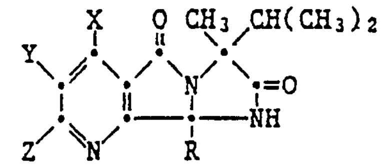

(I)

dans laquelle X, Y et Z représentent chacun, indépendamment les uns des autres, l'hydrogène ou un groupe alkyle en C1—C4, ou bien deux substituants voisins forment ensemble une chaîne alkylène saturée ou alcénylène insaturée à trois ou quatre chaînons qui peut elle-même être substituée par un à quatre groupes alkyle en C1—C4; R représente un groupe nitroalkyle en C1—C4, hydroxyamino, alcoxyamino en C1—C4, alcényloxyamino en C3—C4, alcynyloxyamino en C3—C4 ou —PO(O-alkyle en C1—C4)$_2$, —PO($CH_3$)O-alkyle en C1—C4 ou un groupe hydrazino —$NR_1NR_2R_3$ dans lequel $R_1$ représente l'hydrogène ou un groupe alkyle en C1—C4, $R_2$ représente l'hydrogène, un group alkyle en C1—C4 ou benzyle et $R_3$ représente l'hydrogène, un groupe alkyle en C1—C4, benzoyle, carbamoyle, thiobenzoyle, thiocarbamoyle, mono- ou di-(alkyle en C1—C4)-carbamoyle, mono- ou di-(alkyle en C1—C4)-thiocarbamoyle, anilido, thioanilido, (alcoxy en C1—C4)-carbonyle, (alkyle en C1—C4)-carbonyle, (alkyle en C1—C4)-thiocarbonyle, benzoyloxy, les noyaux phényle pouvant être non substitués ou substitués par des halogènes, des groupes alkyle en C1—C4, halogéno-alkyle en C1—C4 ou alcoxy en C1—C4, ou bien $R_3$ représente un deuxième groupe 3H-imidazo[1',2':1,2]pyrrolo[3,4-b]pyridine-2,5-dione-12-yle ou un groupe triazoylcarbamoyle.

2. Composés selon la revendication 1, dans lesquels R représente le groupe hydrazino, X, Y et Z ayant les significations indiquées dans la revendication 1.

3. La 12-hydrazino-3,8-diméthyl-3-isopropanol-3H-imidazo[1',2':1,2]pyrrolo[3,4-b]pyridine-2,5-dione selon la revendication 1.

4. Composés selon la revendication 1, dans lesquels R représente le groupe hydroxylamino, X, Y et Z ayant les significations indiquées dans la revendication 1.

5. La 12-hydroxylamino-3-isopropyl-3-méthyl-3H-imidazo[1',2':1,2]pyrrolo[3,4-b]pyridine-2,5-dione selon la revendication 1.

6. Composés selon la revendication 1, dans lesquels R représente un groupe alcoxyamino en C1—C4 ou alcényloxyamino en C3—C4, X, Y et Z ayant les significations indiquées dans la revendication 1.

7. La 3-isopropyl-12-méthoxyamino-3-méthyl-3H-imidazo[1',2':1,2]pyrrolo[3,4-b]pyridine-2,5-dione selon la revendication 1.

8. La 12-allyloxyamino-8-n-butyl-3-isopropyl-3-méthyl-3H-imidazo[1',2':1,2]pyrrolo[3,4-b]pyridine-2,5-dione selon la revendication 1.

9. La 12-allyloxyamino-3-isopropyl-3-méthyl-3H-imidazo[1',2':1,2]pyrrolo[3,4-b]pyridine-2,5-dione selon la revendication 1.

10. La 8-éthyl-12-allyloxyamino-3-isopropyl-3-méthyl-3H-imidazo[1',2':1,2]pyrrolo[3,4-b]pyridine-2,5-dione selon la revendication 1.

11. Composés selon la revendication 1, dans lesquels R représente un groupe nitroalkyle en C1—C4, X, Y et Z ayant les significations indiquées dans la revendication 1.

12. La 8-éthyl-3-isopropyl-3-méthyl-12-nitrométhyl-3H-imidazo[1',2':1,2]pyrrolo[3,4-b]pyridine-2,5-dione selon la revendication 1.

13. Procédé de préparation de composés de formule I, revendication 1, caractérisé en ce que l'on fait réagir un composé de formule IIa ou IIb respectivement

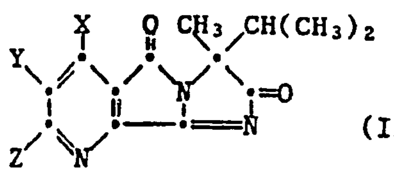

(IIa)

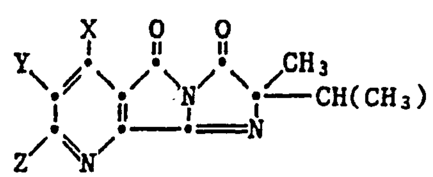

(IIb)

dans lesquelles X, Y et Z ont le significations indiquées dans la revendication 1, dans un solvant ou diluant organique inerte et en présence d'une base, avec un composé de formule III

HR (III)

dans laquelle R a les significations indiquées dans la revendication 1, et on isole du mélange de réaction le produit de condensation formé par acidification à l'aide d'un acide aqueux.

14. Procédé de préparation des composés de formule I, revendication 1, caractérisé en ce que l'on fait d'abord boullir, un composé de formule V

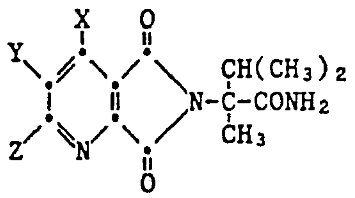

(V)

dans laquelle X, Y et Z ont le significations indiquées dans la revendication 1, dans un solvant ou diluant organique inerte, dans des conditions propres à une déshydratation, puis on fait réagir en présence d'une base avec un composé de formule III

HR (III)

dans laquelle R a l'une des significations indiquées dans la revendication 1, puis on isole du mélange de réaction le produit de condensation formé par acidification à l'aide d'un acide aqueux.

15. Un produit herbicide ou régulateur de la croissance des végétaux, caractérisé en ce qu'il contient en tant que substance active, avec des véhicules et/ou d'autres additifs, au moins un composé selon la revendication 1.

16. L'utilisation d'une substance active selon la revendication 1 ou d'un produit en contenant pour la lutte contre la croissance de végetaux indésirables.

17. L'utilisation d'une substance active selon la revendication 1 ou d'un produit en contenant pour l'inhibition de la croissance des végétaux.

18. L'utilisation d'une substance active selon la revendication 1 ou d'un produit en contenant pour agir sur la croissance des végétaux en vue d'une augmentation de rendement.

19. Procédé pour combattre sélectivement en prélevée ou en post-levée les végétaux adventices dans les cultures de plantes utiles, caractérisé en ce que l'on traite les cultures de plantes utiles ou leur aire de culture par une quantité efficace d'un dérivé d'imidazolinone de formule I, revendication 1, ou d'un produit contenant un tel composé.

20. Procédé pour inhiber la croissance de végétaux au-delà du stade deux feuilles, caractérisé en ce que l'on traite les végétaux pendant leur croissance par une quantité efficace d'un dérivé d'imidazolinone de formule I, revendication 1, ou d'un produit contenant une telle substance active.

## Claims

1. A pyridine compound of formula I

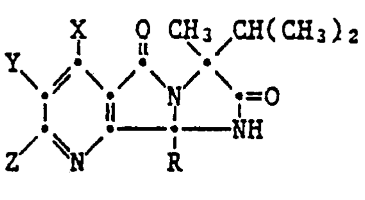

(I)

in which each of X, Y and Z independently of one another is hydrogen or a $C_1$—$C_4$alkyl group, or two adjacent substituents together also form a saturated or unsaturated 3- or 4-membered alkylene chain or alkenylene chain, each of which chains may in turn be substituted by one to four $C_1$—$C_4$alkyl groups, R is a $C_1$—$C_4$nitroalkyl group, the hydroxyamino group, a $C_1$—$C_4$alkoxyamino, $C_3$—$C_4$alkenyloxyamino or $C_3$—$C_4$alkynyloxyamino group, or a —$PO(OC_1$—$C_4alkyl)_2$ or —$PO(CH_3)OC_1$—$C_4$alkyl radical or a hydrazino radical —$NR_1NR_2R_3$, in which $R_1$ is hydrogen or $C_1$—$C_4$alkyl, $R_2$ is hydrogen, $C_1$—$C_4$alkyl or benzyl and $R_3$ is hydrogen, $C_1$—$C_4$alkyl, benzoyl, carbamoyl, thiobenzoyl, thiocarbamoyl, $C_1$—$C_4$alkylcarbamoyl, di($C_1$—$C_4$alkyl)carbamoyl, $C_1$—$C_4$alkylthiocarbamoyl, di($C_1$—$C_4$alkyl)thiocarbamoyl, anilido, thioanilido, $C_1$—$C_4$alkoxycarbonyl, $C_1$—$C_4$alkylcarbonyl, $C_1$—$C_4$alkylthiocarbonyl or benzoyloxy, it being possible for the phenyl rings to be unsubstituted or substited by halogen, $C_1$—$C_4$alkyl, $C_1$—$C_4$haloalkyl or $C_1$—$C_4$alkoxy,

or $R_3$ is a second 3H-imidazo[1',2':1,2]pyrrolo[3,4-b]pyridine-2,5-dione-12-yl radical or a triazolylcarbamoyl radical.

2. A compound according to claim 1, in which R is the hydrazino radical, and X, Y und Z are as defined in claim 1.

3. 12-Hydrazino-3,8-dimethyl-3-isopropyl-3H-imidazo[1',2':1,2]pyrrolo[3,4-b]pyridine-2,5-dione according to claim 1.

4. A compound according to claim 1, in which R is the hydoxylamino radical, and X, Y and Z are as defined in claim 1.

5. 12-Hydroxylamino-3-isopropyl-3-methyl-3H-imidazo[1',2':1,2]pyrrolo[3,4-b]pyridine-2,5-dione according to claim 1.

6. A compound according to claim 1, in which R is a $C_1$—$C_4$alkoxyamino or $C_3$—$C_4$alkenyloxyamino radical and X, Y and Z are as defined in claim 1.

7. 3-Isopropyl-12-methoxyamino-3-methyl-3H-imidazo[1',2':1,2]pyrrolo[3,4-b]pyridine-2,5-dione according to claim 1.

8. 12-Allyloxyamino-8-n-butyl-3-isopropyl-3-methyl-3H-imidazo[1',2':1,2]pyrrolo[3,4-b]pyridine-2,5-dione according to claim 1.

9. 12-Allyloxyamino-3-isopropyl-3-methyl-3H-imidazo[1',2':1,2]pyrrolo[3,4-b]pyridine-2,5-dione according to claim 1.

10. 8-Ethyl-12-Allyloxyamino-3-isopropyl-3-methyl-3H-imidazo[1',2':1,2]pyrrolo[3,4-b]pyridine-2,5-dione according to claim 1.

11. A compound according to claim 1, in which R is a $C_1$—$C_4$nitroalkyl radical, and X, Y and Z are as defined in claim 1.

12. 8-Ethyl-3-isopropyl-3-methyl-12-nitromethyl-3H-imidazo[1',2':1,2]pyrrolo[3,4-b]pyridine-2,5-dione according to claim 1.

13. A process for the preparation of a compound of formula I, claim 1, which process comprises reacting a compound of formula IIa or IIb

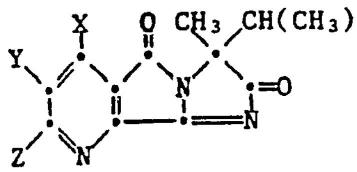

(IIa)

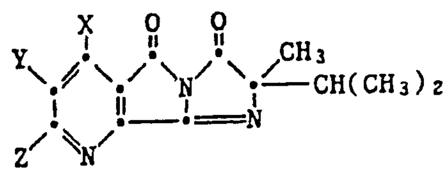

(IIb)

in which X, Y and Z are as defined in claim 1, in an inert organic solvent or diluent and in the presence of a base, with a compound of formula III

HR (III)

in which R is as defined in claim 1, and isolating the resultant condensation product from the reaction mixture by acidification with an aqueous acid.

14. A process for the preparation of a compound of formula I, claim 1, which process comprises first boiling a compound of formula V

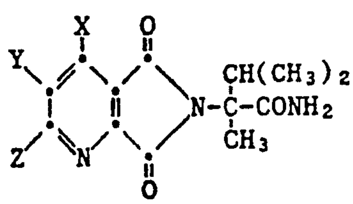

(V)

in which X, Y and Z are as defined in claim 1, in an inert organic solvent or diluent and under dehydrating conditions, then reacting the resultant compound, in the presence of a base, with a compound of formula III

HR (III)

in which R is as defined in claim 1, and isolating the resultant condensation product from the reaction mixture by acidification with an aqueous acid.

15. A herbicidal and plant growth regulating composition which contains, as active ingredient, at least one compound according to claim 1, together with carriers and/or other adjuvants.

16. The use of an active ingredient according to claim 1, or of a composition containing such a compound, for controlling undesired plant growth.

17. The use of an active ingredient according to claim 1, or of a composition containing such a

compound, for inhibiting plant growth.

18. The use of an active ingredient according to claim 1, or of a composition containing such a compound, for influencing plant growth for the purpose of increasing the yield.

19. A method of selectively controlling weeds pre- or postemergence in crops of useful plants, which method comprises treating the crops of useful plants or the crop areas thereof with an effective amount of an imidazolinone compound of formula I, claim 1, or of a composition containing such a compound.

20. A method of suppressing plant growth beyond the 2-leaf stage, which method comprises treating the plants during their growth with an effective amount of an imidazolinone compound of formula I, claim 1, or of a composition containing such an active ingredient.